(19) Europäisches Patentamt
European Patent Office
Office européen des brevets

(11) **EP 4 400 831 A1**

(12) **EUROPEAN PATENT APPLICATION**
published in accordance with Art. 153(4) EPC

(43) Date of publication:
**17.07.2024 Bulletin 2024/29**

(51) International Patent Classification (IPC):
**G01N 21/49** (2006.01)  **G01N 21/47** (2006.01)
**G01N 21/45** (2006.01)  **G01N 33/18** (2006.01)
**G01N 21/51** (2006.01)

(21) Application number: **22865065.1**

(52) Cooperative Patent Classification (CPC):
**G01N 33/1826; G01N 15/06; G01N 21/031;**
**G01N 21/45; G01N 21/51; G01N 33/18;**
G01N 21/94; G01N 2021/479

(22) Date of filing: **01.09.2022**

(86) International application number:
**PCT/KR2022/013103**

(87) International publication number:
**WO 2023/033563 (09.03.2023 Gazette 2023/10)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **06.09.2021 KR 20210118254**

(71) Applicant: **The Wave Talk, Inc.**
**Daejeon 34051 (KR)**

(72) Inventors:
• **KIM, Young Dug**
  **Seongnam-si Gyeonggi-do 13597 (KR)**
• **CHO, Kyoung Man**
  **Seoul 06224 (KR)**
• **CHEON, Doo Young**
  **Uijeongbu-si Gyeonggi-do 11769 (KR)**

(74) Representative: **AWA Sweden AB**
**Box 45086**
**104 30 Stockholm (SE)**

(54) **WATER QUALITY INSPECTION DEVICE**

(57)      An embodiment of the present disclosure provides a water examination device including: a main body; a fluid accommodation unit formed in the main body; a wave source for irradiating waves toward the fluid accommodation unit; a detector for detecting a laser speckle in time series, the laser speckle being generated due to multiple scattering of the waves in the fluid; a controller for estimating existence of foreign material in the fluid in real-time by using the detected laser speckle, wherein an imaginary first plane passing through the wave source is parallel to an imaginary second plane passing through the detector.

FIG. 2

**Description**

Technical Field

One or more embodiments of the present disclosure relate to a water examination device.

Background Art

In general, a fluid such as water or beverage is supplied to a user through various processes, e.g., filtration, etc. A fluid for drinking purposes has to be supplied to the user after materials, e.g., microbes, other than additives that are added in the fluid according to necessity, are removed therefrom. However, during a process of treating the fluid, microbes in the fluid may unintentionally proliferate under a circumstance in which the fluid is in contact with external air.
Various methods have been suggested in order to examine water by sensing microbes in the fluid, but it is very difficult to sense a minimum number of microbes in the fluid.

Detailed Description of the Invention

Technical Problem

The present disclosure provides a water examination device which examines water by sensing microbes in a fluid in real-time by using a chaotic wave sensor.

Technical Solution to Problem

An embodiment of the present disclosure provides a water examination device including: a main body; a fluid accommodation unit formed in the main body; a wave source for irradiating waves toward the fluid accommodation unit; a detector for detecting a laser speckle in time series, the laser speckle being generated due to multiple scattering of the waves in the fluid; a controller for estimating existence of foreign material in the fluid in real-time by using the detected laser speckle, wherein an imaginary first plane passing through the wave source is parallel to an imaginary second plane passing through the detector.

Advantageous Effects of Disclosure

A water examination device according to an embodiment of the present disclosure examines water by estimating whether there are microbes in a fluid and/or a concentration rapidly at low costs, according to a change in a temporal correlation or a spatial correlation of a laser speckle.

Brief Description of Drawings

FIG. 1 is a conceptual diagram showing a water monitoring system according to an embodiment of the present disclosure.
FIG. 2 is a conceptual diagram showing a water examination device in the water monitoring system of FIG. 1.
FIG. 3 is a diagram for describing principles of a chaotic wave sensor according to an embodiment of the present disclosure.
FIG. 4 is a perspective view of a water examination device actually implementing the conceptual diagram of FIG. 2.
FIG. 5 is a cross-sectional view taken along line A-A' of FIG. 4.
FIG. 6 is a diagram provided to explain a structure for irradiating from a wave source to the inside of a fluid accommodation unit.
FIG. 7 is a diagram provided to explain a structure in which a laser speckle emitted from the inside of a fluid is provided to a detector.
FIG. 8 is a diagram provided to explain the positional relationship between a first through hole and a second through hole.
FIG. 9 is a diagram provided to explain a process of detecting microbes by placing a cup on a fluid accommodation unit.
FIGS. 10 and 11 are diagrams provided to describe a water examination device according to another embodiment of the present disclosure.
FIG. 12 is a cross-sectional view taken along line B-B' of FIG. 4.

FIG. 13 is a plan view of the water examination device of FIG. 4.

FIG. 15 is a diagram illustrating the use of a water examination device.

FIG. 16 is a graph of a temporal correlation coefficient according to a separation gap between a wave source and a detector, obtained by using the water examination device according to an embodiment of the present disclosure.

FIG. 17 is a table listing temporal correlation coefficients according to concentrations according to the separation gap between the wave source and the detector.

FIGS. 18A to 18C are graphs showing a temporal correlation coefficient according to a bacterial concentration in a fluid in a water examination device according to an embodiment of the present disclosure.

FIGS. 19 and 20 are diagrams for describing a principle of detecting microbes in a water examination device according to another embodiment of the present disclosure.

FIG. 21 is a flowchart illustrating a water monitoring method according to an embodiment of the present disclosure.

FIG. 22 is a flowchart illustrating operation S120 of FIG. 21 in more detail.

Best Mode

An embodiment of the present disclosure provides a water examination device including: a main body; a fluid accommodation unit formed in the main body; a wave source for irradiating waves toward the fluid accommodation unit; a detector for detecting a laser speckle in time series, the laser speckle being generated due to multiple scattering of the waves in the fluid; a controller for estimating existence of foreign material in the fluid in real-time by using the detected laser speckle, wherein an imaginary first plane passing through the wave source is parallel to an imaginary second plane passing through the detector.

In an embodiment of the present disclosure, the imaginary first plane may pass through an irradiation surface on which the wave source irradiates the wave, and the imaginary second plane may pass through a detection plane of the detector.

In an embodiment of the present disclosure, a distance between the imaginary first plane and the imaginary second plane may be greater than a diameter of the wave irradiated from the wave source.

The fluid accommodation unit may include: a bottom portion formed in the main body; and a wall portion formed to have a certain angle with respect to the bottom portion.

In an embodiment of the present disclosure, a certain region of an inner surface of the wall portion to which the waves are irradiated may include a light-absorbing material.

In an embodiment of the present disclosure, the certain area to which the wave is irradiated may be larger than a diameter of the wave.

In an embodiment of the present disclosure, the inner surface of the wall portion may be entirely formed of a light-absorbing material.

In an embodiment of the present disclosure, the inner surface of the bottom portion may be entirely formed of a light-absorbing material.

In an embodiment of the present disclosure, the fluid accommodation unit may include a first through hole corresponding to the location of the wave source and through which the wave passes, and a second through hole corresponding to the location of the detector.

In an embodiment of the present disclosure, the imaginary first plane may pass through the first through hole, and may not pass through the second through hole.

In an embodiment of the present disclosure, the diameter of the first through hole may be smaller than the diameter of the second through hole.

In an embodiment of the present disclosure, the controller may obtain the temporal correlation of the laser speckle by using the detected laser speckle, and estimate existence of microbes in the fluid in real-time based on the temporal correlation.

In an embodiment of the present disclosure, the temporal correlation may include a difference between first image information of the laser speckle detected at a first time and second image information of the laser speckle detected at a second time that is different from the first time.

In an embodiment of the present disclosure, the first image information and the second image information may each include at least one of pattern information of the laser speckle and intensity information of the wave.

In an embodiment of the present disclosure, the controller may obtain a spatial correlation of an interference pattern in an optical image detected by the detector and determine existence of a foreign material in the fluid based on a change in the spatial correlation of the interference pattern over time.

Other aspects, features and advantages of the disclosure will become better understood through the accompanying drawings, the claims and the detailed description.

Mode of Disclosure

Fine scattering may occur in a fluid L.

Waves in the fluid L may be less multi-scattered than a case where there is a homogeneous material, and due to these scattering, the waves cause constructive interference or destructive interference with each other, and laser speckle may be detected by a detector 120. In this case, when there is no foreign material in the fluid L, the degree of scattering of waves by atoms or molecules of the fluid L may be constant on average as long as there is no external stimulus. Here, the external stimulus may refer to generation of bubbles in the fluid L due to vibration or impact.

In a water examination device 100, the path of the waves may be slightly changed due to the movement of the microbes when the microbes M exist in the fluid L contained in a cup 190. The fine change in the wave path may generate a change in the speckle pattern, and accordingly, the temporal change in the speckle pattern may be measured to rapidly detect whether there are the microbes M in the fluid L.

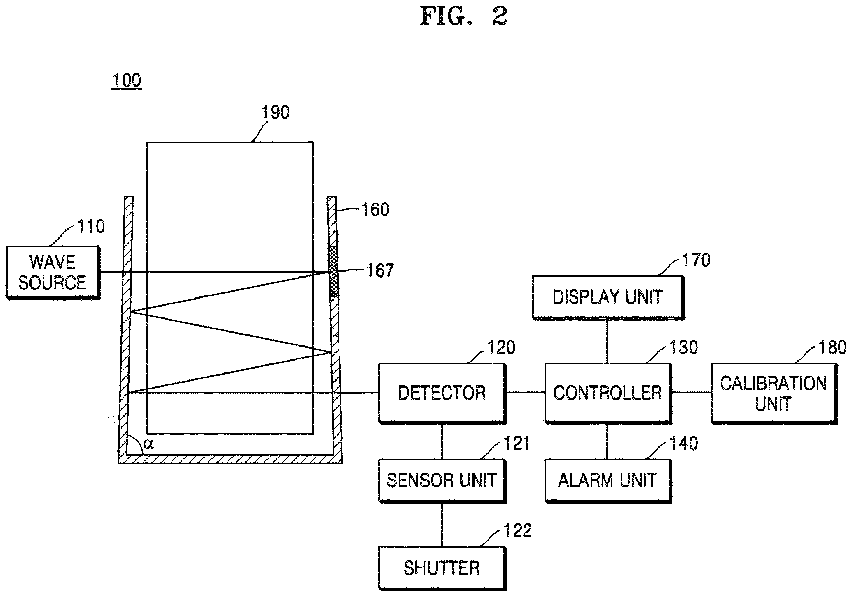

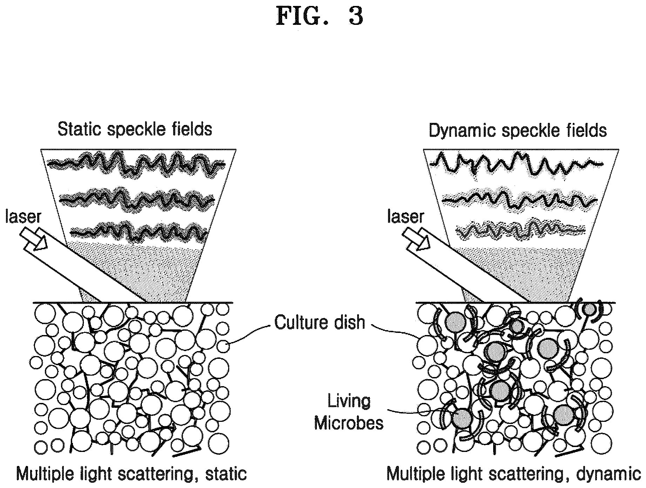

Referring back to FIG. 2 and FIG. 3, the water examination device 100 according to an embodiment of the present disclosure may include a wave source 110, a detector 120, and a controller 130.

The wave source 110 may irradiate a wave toward the fluid L in the cup 190 accommodated in a fluid accommodation unit 160. The wave source 110 may include all kinds of source devices capable of generating waves, and may be, for example, a laser irradiating light of a certain wavelength band. Although the present disclosure is not limited to the kind of wave source, for convenience of description, a case where the wave source is a laser will be described below.

For example, the laser having excellent coherence may be used as the wave source 110 in order to form speckles on the fluid L. Here, when a spectral bandwidth of the wave source is shorter, a measuring accuracy may increase, wherein the spectral bandwidth determines the coherence of the laser wave source. That is, when a coherence length increases, the measuring accuracy also increases. Accordingly, a wave source irradiating the laser having a spectral bandwidth that is less than a reference bandwidth set in advance may only be used as the wave source 110, and the shorter the spectral bandwidth is as compared with the reference bandwidth, the higher the measuring accuracy is. For example, the spectral bandwidth of the wave source may be set to satisfy following condition of Inequality 1 below.

[Inequality 1]

$$Spectral\ bandwidth < 5nm$$

According to Inequality 1 above, when the light is irradiated into the fluid at every reference time in order to measure a variation in the laser speckle pattern, the spectral bandwidth of the wave source 110 may be maintained to be less than 5 nm.

The detector 120 may detect in time series the laser speckles that are generated when the irradiated waves are multiple scattered in the fluid L. In an embodiment, the detector 120 may detect the laser speckles that are generated when the irradiated waves are multiple scattered in the fluid L, at every preset time. Here, the time may denote one instant during continuous flow of time, and times may be set in advance with constant time intervals therebetween, but are not limited thereto, that is, may be set in advance with an arbitrary time interval.

The detector 120 may include a sensing unit corresponding to the kind of the wave source 110, for example, a CCD camera that is an imaging device in a case where a light source of a visible ray wavelength band is used. The detector 120 may detect the laser speckle at a first time at least, and may detect the laser speckle at a second time, and then, may provide the controller 130 with the detected laser speckles. The first time and the second time are just examples selected for convenience of description, and the detector 120 may detect laser speckles at a plurality of times more than the first and second times.

In detail, when the wave is irradiated to the fluid L, the incident wave may generate laser speckle due to the multiple scattering. The laser speckle is generated by light interference effect, and thus, when there are no microbes in the fluid L, a constant interference pattern may be shown on average over time. That is, even when the fluid L has fluidity, if no foreign material exists thereinside, the laser speckle may exhibit a constant interference pattern on average due to the unique characteristics of the fluid L.

Comparing with this, when microbes exist in the fluid L, the laser speckle may vary over time compared to the constant interference pattern on average due to the movement of the microbes M. The detector 120 detects the laser speckle, which varies over time, in time series to provide the same to the controller 130. The detector 120 may detect the laser speckle at a sufficient speed to sense the movement of the microbes M, for example, 25 frames to 30 frames per second.

In addition, when the image sensor is used as the detector 120, the image sensor may be arranged so that a size d of one pixel in the image sensor is equal to or less than a grain size of the speckle pattern. For example, the image sensor may be arranged in the optical system included in the detector 120, to satisfy the condition of Inequality

2 below.

[Inequality 2]

$$d \leq speckle \ grain \ size$$

As expressed by Inequality 2 above, the size d of one pixel in the image sensor has to be equal to or less than the grain size of the speckle pattern, but the size of the pixel is too small, an undersampling may occur and it may be difficult to utilize the pixel resolution. Accordingly, in order to achieve an effective signal to noise ratio (SNR), the image sensor may be arranged to make five or less pixels correspond to the speckle grain size.

The controller 130 may obtain a temporal correlation of the laser speckle, by using the detected laser speckle. The controller 130 may estimate whether the microbes exist in the fluid L in real-time based on the obtained temporal correlation. The term "real-time" used herein refers to estimating the presence or absence of microbes M within 10 seconds, or estimating the presence or absence of microbes M within 3 seconds, or estimating the presence or absence of microbes M within 1 second.

In an embodiment, the controller 130 may estimate whether the microbes exist by using a difference between first image information of the laser speckle detected at a first time and second image information of the laser speckle detected at a second time that is different from the first time. Here, the first image information and the second image information may include at least one of laser speckle pattern information and wave intensity information.

In addition, according to the embodiment, the difference between the first image information at the first time and the second image information at the second time is not only used, but image information of a plurality of laser speckles at a plurality of times may be also used. The controller 130 may calculate a temporal correlation coefficient between the images by using image information of the laser speckles generated at the plurality of times set in advance, and may estimate existence of the microbes M in the fluid L based on the temporal correlation coefficient. The temporal correlation coefficient between the detected laser speckle images may be calculated by using Inequality 3 below.

[Equation 3]

$$\bar{C}(x,y;\tau) = \frac{1}{T-\tau}\sum_{t=1}^{T-\tau}\bar{I}(x,y;t)\bar{I}(x,y;t+\tau)\delta t$$

Regarding Equation 3, $C$ is a temporal correlation coefficient, $I$ is the normalized light intensity, $(x,y)$ is the pixel coordinates of the camera, t is the measured time, T is the total measurement time, and $\tau$ is a time lag.

According to Equation 3, the temporal correlation coefficient may be calculated, and as an embodiment, the existence of the microbes may be estimated by analyzing whether the temporal correlation coefficient is below a reference value set in advance. In detail, when the temporal correlation coefficient is below the reference value beyond an error range set in advance, it may be estimated that the microbes exist.

In addition, the detector 120 may estimate the concentration of a foreign material in the fluid L contained in the cup 190. Here, the detector 120 may perform a function of measuring a turbidity of the fluid L by estimating the concentration of the foreign material in the fluid L. It is difficult to measure the foreign material concentration of 105 cfu/ml or less by using a general turbidity measuring device. However, the detector 120 according to an embodiment of the present disclosure may measure the foreign material concentration of 105 cfu/ml or less through a method of determining the foreign material concentration as described below. Here, the foreign material is not limited to the microbes. Hereinafter, for convenience of description, a method of determining the concentration of the microbes by using the laser speckles in the controller 130 will be described based on an example in which the foreign material is a microbe.

The controller 130 may calculate a standard deviation of light intensity of the laser speckle, with respect to a laser speckle image captured at every reference time. As the microbes existing in the fluid L continuously move, constructive interference and destructive interference may vary according to the movements. Here, when the constructive interference and the destructive interference change, the light intensity may largely change. Then, the controller 130 may calculate the standard deviation representing the variation degree of the light intensity, to detect the microbes in the cup 190 and measure a distribution degree of the microbes.

For example, the controller 130 combines the laser speckle images that are measured at every time determined in advance, and may calculate the standard deviation of the light intensity of the laser speckle over time in the combined images. The standard deviation of the light intensity of the laser speckle over time may be calculated by using Inequality

4 below.

[Equation 4]

$$S(x, y) = \sqrt{\frac{1}{T} \sum_{t=1}^{T} (I_t(x, y) - \bar{I})^2}$$

Regarding Equation 4, S is standard deviation, (x,y) is camera pixel coordinates, T is total measurement time, t is measurement time, It is intensity of light measured at time t, and $I$ is the average light intensity over time.

The constructive and destructive interferences may vary depending on the movements of the microbes, and the standard deviation value calculated according to the Inequality 4 increases. Thus, the concentration of the microbes may be measured based on the standard deviation value. However, one or more embodiments of the present disclosure are not limited to the method of measuring the concentration of microbes by using Equation 4 above, and the concentration of microbes may be measured by any method provided that the method uses the difference in the detected laser speckles.

In addition, the controller 130 may measure distribution, that is, concentration of the microbes included in the fluid, based on a linear relationship between a magnitude of the standard deviation value of the laser speckle light intensity and the concentration of the microbes.

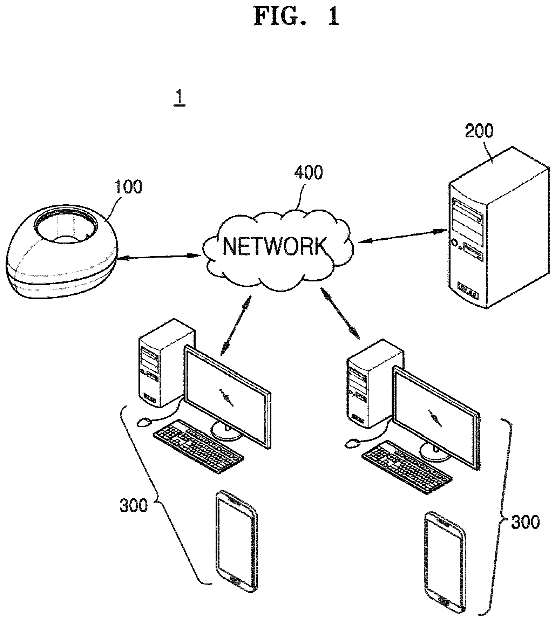

Referring back to FIG. 2, the water examination device 100 according to an embodiment of the present disclosure may further include an alarm unit 140 and the display unit 170. In addition, the water examination device 100 may be connected to an external user terminal 300 (see FIG. 1) or a server 200 (see FIG. 1) via a network 400 (see FIG. 1).

When a signal representing that there are microbes is input from the controller 130, the alarm unit 140 may notify the user. The alarm unit 140 may notify that there are microbes in the fluid by using any one of sound and light. The alarm unit 140 may include an illumination unit such as an LED generating an alarm signal via light and a speaker (not shown) generating an alarm signal via sound, and the light and the sound may be simultaneously generated.

Also, the water examination device 100 may further include a communication unit (not shown) for communicating with the user terminal 300 (see FIG. 1). When the signal representing that there are the microbes is input from the controller 130, the alarm unit 140 may provide the user terminal 300 (see FIG. 1) with the information including a microbe sensing signal via a wired or wireless communication unit (not shown). Also, the alarm unit 140 may provide the server 200 (see FIG. 1) with the above information.

When information about whether to sense the microbes, the time of sensing the microbes, and the concentration of the microbes is uploaded through the alarm unit 140, the water examination device 100 registers the information on the server 300 (see FIG. 1) and provides an interface for allowing other users to search for data registered on the server 300 (see FIG. 1). The water examination device 100 according to an embodiment may establish a database including microbes generation situation, etc. through the above processes. The user terminal 300 (see FIG. 1) may include a personal computer or a portable terminal through which Web service may be used in wired/wireless communication environment.

The display unit 170 outputs a detecting result from the detector 120 as visual information. That is, various data may be output by processing information on existence of the microbes in the fluid L and/or concentration of the microbes, and turbidity information in the fluid estimated therefrom, etc. Here, image treatment processes for outputting received image as an image through the display unit 170 may be performed by the controller 130.

Here, the water examination device 100 of the water monitoring system 1 according to an embodiment of the present disclosure may further include a calibration unit 180.

The calibration unit 180 controls the water examination device 100 such that the intensity of light irradiated from the wave source 110 and measured by the detector 120 after passing through the inside of the fluid L is within a certain range set in advance.

Here, according to an embodiment of the present disclosure, the calibration unit 180 may control a shutter speed of the detector 120 such that the intensity of light measured by the detector 120 may be within a certain range set in advance. This will be described below in more detail.

The detector 120 is formed similarly to a camera and may detect the light that is irradiated from the wave source 110 and passes through the fluid L. The detector 120 may include a sensor unit 121 including an image sensor and a shutter 122. Basically, when the shutter 122 is opened, the light is incident in the sensor unit 121, and when the shutter 122 is closed, the light incident in the sensor unit 121 is blocked. Here, the shutter speed denotes a speed of opening and closing the shutter 122, and when the shutter speed is slow, the intensity of light incident in the sensor unit 121 increases, and when the shutter speed is fast, the intensity of light incident in the sensor unit 121 decreases.

Here, a calibration reference value may be determined from an average of intensity values of respective pixels in an image detected by the detector 120.

In detail, in case of an 8-bits image sensor, an intensity of each pixel has a value between 0 to 255, and an intermediate value, that is, 128, may be set as a reference value. In addition, based on various experimental data, an appropriate light intensity range may be set as ±5 from the reference value. In addition, a minimum value of the appropriate light intensity range is set as a first reference value and a maximum value of the appropriate light intensity range may be set as a second reference value.

For example, when the average value of the intensities ranges from 123 to 133, it may be set as an appropriate light intensity. (Here, the first reference value may be 123 and the second reference value may be 133.) In addition, an average of the intensity values of the pixels in the image that is irradiated from the wave source 110 and detected by the detector 120 after passing through the fluid L is measured, and when the average of the intensity values is less than 123, the calibration unit 180 determines that the light intensity is not sufficient and may decrease the shutter speed in order to increase the intensity of light entering the sensor unit 121. On the contrary, when the average of the intensity values of the pixels in the detected image is greater than 133, the calibration unit 180 determines that the light intensity is excessive and may increase the shutter speed in order to decrease the intensity of the light incident in the sensor unit 121.

As described above, because the intensity of light detected by the detector 120 is calibrated within a certain rage by the calibration unit 180, a consistent reference value may be set with respect to the intensities of the light measured from a plurality of water examination devices 100 located at different positions.

As described above, after calibrating the intensity of light detected by the detector 120 within a certain range by using the calibration unit 180, the controller 130 may obtain the temporal correlation of the laser speckle. Thus, even when the performance of the wave source 110 degrades or there is a deviation in container containing the fluid sample, an error in obtaining the temporal correlation of the laser speckle may be minimized.

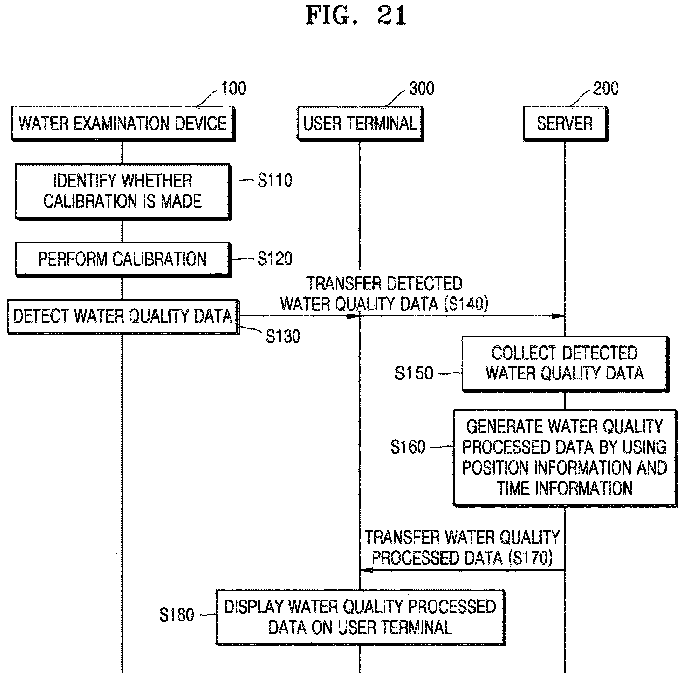

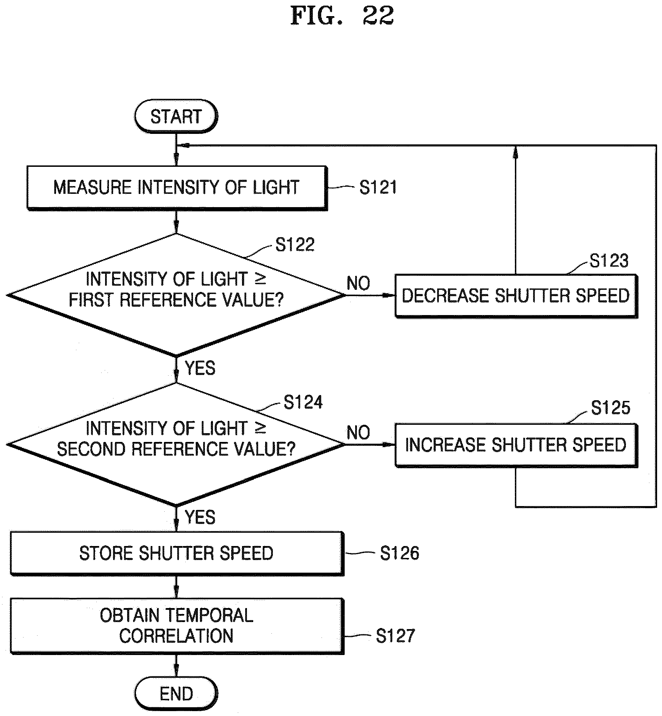

A method of performing the water quality monitoring by using the water monitoring system 1 according to an embodiment of the present disclosure will be described below with reference to FIGS. 21 and 22.

In addition, according to another embodiment of the present disclosure, the calibration unit 180 controls the intensity of light irradiated from the wave source 110 itself, in order to set the intensity of light measured by the detector 120 within a certain range.

That is, an average of the intensity values of the pixels in the image that is irradiated from the wave source 110 and detected by the detector 120 after passing through the fluid L is measured, and when the average of the intensity values is less than 123, the calibration unit 180 determines that the light intensity is not sufficient and may increase the intensity of light irradiated from the wave source 110 by increasing the voltage of the wave source 110 in order to increase the intensity of the light incident in the sensor unit 121. On the contrary, when the average of the intensity values of the pixels in the detected image is greater than 133, the calibration unit 180 determines that the light intensity is excessive and may decrease the intensity of light irradiated from the wave source 110 by decreasing the voltage of the wave source 110 in order to decrease the intensity of the light incident in the sensor unit 121.

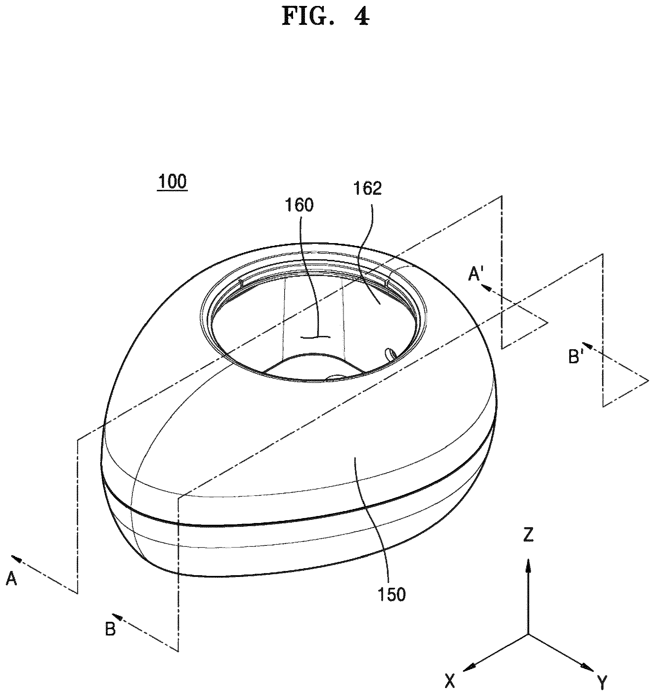

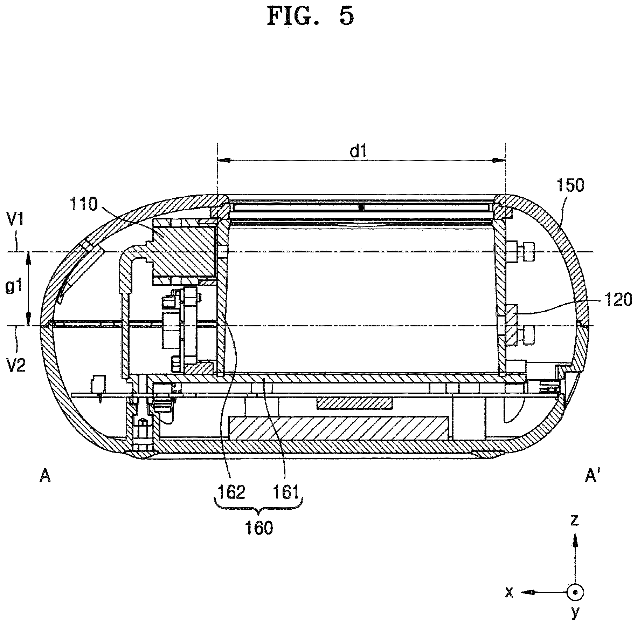

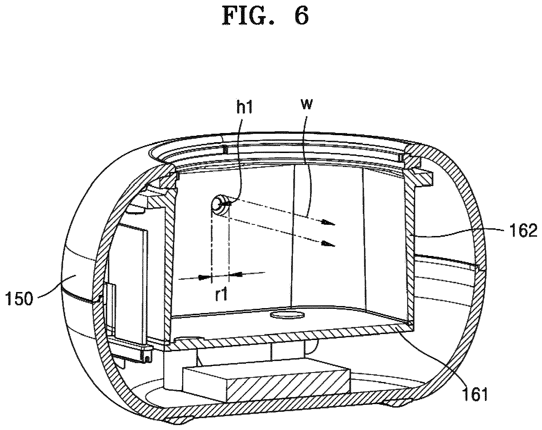

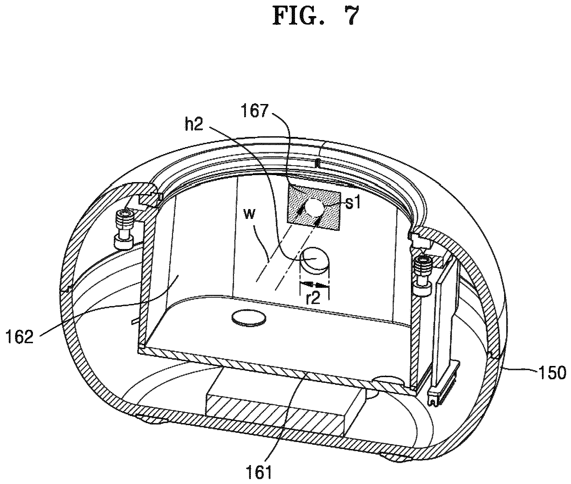

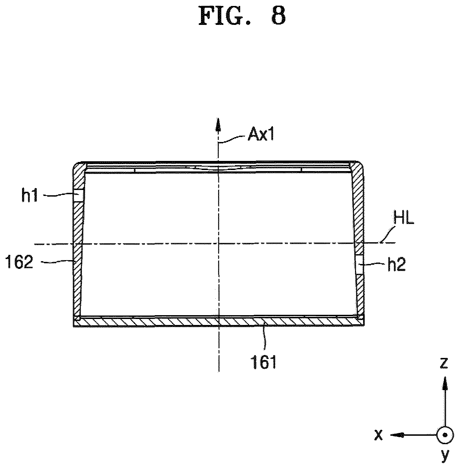

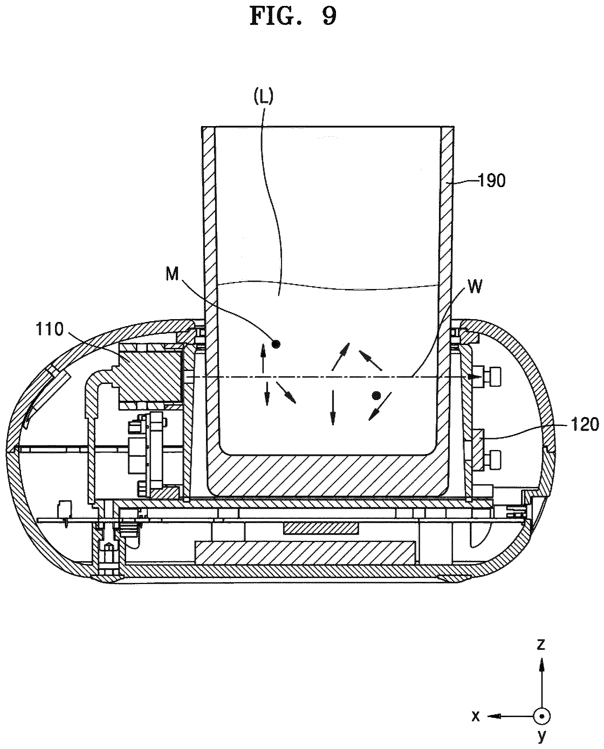

FIG. 4 is a perspective view of a water examination device actually implementing the conceptual diagram of FIG. 2, and FIG. 5 is a cross-sectional view taken along line A-A' of FIG. 4. FIG. 6 is a diagram provided to explain a structure for irradiating from a wave source to the inside of a fluid accommodation unit, and FIG. 7 is a diagram provided to explain a structure in which a laser speckle emitted from the inside of a fluid accommodation unit is provided to a detector. FIG. 8 is a diagram provided to explain the positional relationship between a first through hole and a second through hole, and FIG. 9 is a diagram provided to explain a process of detecting microbes by placing a cup on a fluid accommodation unit.

Referring to FIGS. 4 to 9, the water examination device 100 according to an embodiment of the present disclosure may include the wave source 110, the detector 120, a main body 150, and the fluid accommodation unit 160. Also, although not shown in FIGS. 4 to 8, the water examination device 100 may further include the controller 130 (see FIG. 2) and the alarm unit 140 (see FIG. 2) described above with reference to FIG. 2. Here, the wave source 110 and the detector 120 are described above with reference to FIG. 2, and detailed descriptions thereof are omitted.

The main body 150 forms an outer appearance of the water examination device 100, and may include the wave source 110, the detector 120, and the fluid accommodation unit 160 formed therein. Referring to the related diagram, the main body 150 forms an eccentric streamline (or an oval shape when viewed from above) as a whole, and the fluid accommodation unit 160 into which the cup 190 can be inserted from the top to the inside may be formed. However, the concept of the present disclosure is not limited thereto, and the size, shape, and material of the main body 150, and the position where the fluid accommodation unit 160 is formed in the main body 150 may vary. Here, the fluid accommodation unit 160 may function as a cup accommodation unit in which the cup 190 may be accommodated.

The fluid accommodation unit 160 may be formed such that the cup 190 may be inserted to inside (that is, toward the center portion) from the upper portion of the main body 150. The fluid accommodation unit 160 may include a bottom portion 161 and a wall portion 162. In other words, it may be expressed that the bottom portion 161 and the wall portion 162 may form the fluid accommodation unit 160 in which the cup 190 may be accommodated and seated.

The bottom portion 161 forms a bottom surface of the fluid accommodation unit 160 and has a flat surface.

The wall portion 162 may be formed in a substantially vertical direction from the bottom portion 161, and may surround an edge of the bottom portion 161 to form an accommodation space for accommodating the cup 190 therein. The wall portion 162 may cover the bottom portion 161, with an annular structure to accommodate the circular cup 190. However, the spirit of the present disclosure is not limited thereto, and may be formed in a shape corresponding to the shape of the cup 190.

In an embodiment, the wave source 110 and the detector 120 may be disposed outside the fluid accommodation unit 160. At this time, an imaginary first plane V1 passing through the wave source 110 may be disposed parallel to an imaginary second plane V2 passing through the detector 120. Here, the imaginary first plane V1 may pass perpendicularly to the irradiation surface on which the wave source 110 irradiates wave w, and the imaginary second plane V2 may pass perpendicularly to the detection plane of the detector 120.

In an embodiment, the wave source 110 and the detector 120 may be disposed at different positions in the z-direction upward from the bottom portion 161 of the fluid accommodation unit 160. Through this, the wave source 110 and the detector 120 may have a constant separation gap g1 in the z direction, and the wave w irradiated from the wave source 110 may not be directly incident to the detector 120.

When the wave w irradiated from the wave source 110 is directly incident to the detector 120, since the center wave having the largest intensity and passing through the center of the path of the wave w is incident to the detector 120, the detector 120 may not detect a laser speckle generated by fine scattering. According to the present disclosure, however, even a fine laser speckle can be accurately detected by separating the wave source 110 and the detector 120 by a certain distance.

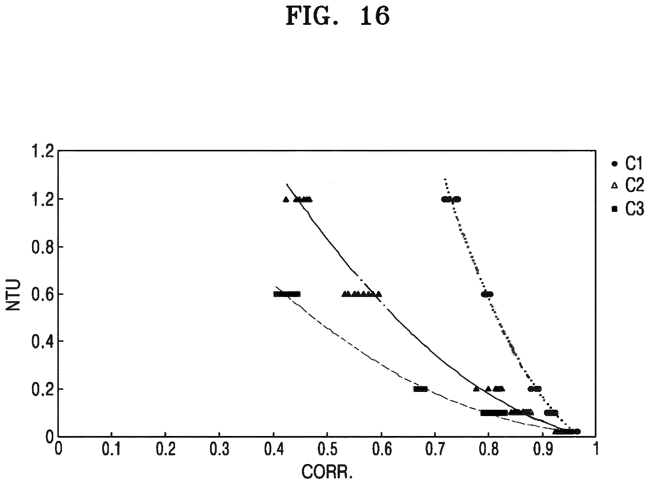

At this time, the separation gap g1 between the wave source 110 and the detector 120 may affect detection power for detecting the existence of microbes. As will be described later with reference to FIG. 16, the shorter the separation gap g1 between the wave source 110 and the detector 120 is, the better the detection ability of detecting the existence of microbes is. Specifically, the shorter the separation gap g1 between the wave source 110 and the detector 120 is, the more effectively fine scattered lights generated on the path through which the wave w passes may be detected, and the range of change in the temporal correlation coefficient of the detected laser speckle may be increased. When the range of change in the temporal correlation coefficient of the detected laser speckle is increased, the water examination device 100 may more clearly distinguish different concentrations of microbes M, enabling sensitive detection of low concentrations of microbes M.

However, the separation gap g1 between the wave source 110 and the detector 120, that is, the interval between the first plane V1 and the second plane V2 may be greater than the diameter s1 of the wave w irradiated from the wave source 110. When the distance between the first plane V1 and the second plane V2 is smaller than the diameter s1 of the wave, a part of the wave w irradiated from the wave source 110 is directly incident on the detector 120 and thus, the detection of laser speckle may be inhibited. Accordingly, the separation gap g1 of the wave source 110 and the detector 120 may be designed to be larger than the diameter s1 of the wave w.

As illustrated in FIG. 7, the inner surface of the wall portion 162 may have a certain area 167 which is formed of a light-absorbing material and to which the wave w is irradiated. As the wave w irradiated from the wave source 110 arrives at the inner surface of the wall portion 162 and is reflected, the detection by the detector 120 may be affected. According to the present disclosure, since at least the certain area 167 of the inner surface, to which the wave w is irradiated is formed of a light-absorbing material, the incident of the wave w which is reflected to the detector 120 may be reduced. Here, the area of a certain area 167 to which the wave w is irradiated, may be greater than the diameter s1 of the wave w.

In an embodiment, a black adhesive film may be attached to the certain area 167 of the inner surface of the wall portion 162. In an embodiment, a light-absorbing layer may be coated on the certain area 167 of the inner surface of the wall portion 162, and the wall portion 162 itself may include a light-absorbing material. The light-absorbing material may be a material that absorbs waves of all wavelength bands without reflecting the same, or may include materials that absorb waves of a specific wavelength band. For example, the light-absorbing material may include metal and two or more alloy oxides of copper oxide (CuOx), nickel oxide (NiOx), molybdenum oxide (MoOx), or copper/nickel/molybdenum.

In an embodiment, the inner surface of the wall portion 162 may be completely formed of a light-absorbing material. Alternatively, in addition to the wall portion 162 of the fluid accommodation unit 160, the inner surface of the bottom portion 161 may include a light-absorbing material. That is, the inner surface of the fluid accommodation unit 160 is completely formed of a light-absorbing material, and thus, waves irradiated from the wave source 110 into the fluid accommodation unit 160 may not be scattered due to factors other than materials in the fluid L. As a result, although the intensity of the laser speckle detected by the detector 120 is weakened, according to the present disclosure, information on the laser speckle generated by the material in the fluid L may be more focused during the detection.

Meanwhile, referring to FIGS. 6 and 7, in order for the wave source 110 to irradiate waves into the fluid accommodation unit 160, a first through hole h1 may be formed in an area of the fluid accommodation unit 160 corresponding to the wave source 110. Likewise, in order for the detector 120 to detect the laser speckle emitted from the fluid accommodation unit 160, a second through hole h2 may be formed in an area of the fluid accommodation unit 160 corresponding to the detector 120.

Here, since the imaginary first plane V1 passing through the wave source 110 is disposed parallel to the imaginary second plane V2, the imaginary first plane V1 may pass through the first through hole h1 and not pass through the second through hole h2. In an embodiment, a diameter r1 of the first through hole h1 may be smaller than the diameter r2 of the second through hole h2.

The shorter the separation gap g1 of the detector 120 and the wave source 110 is, the greater the detection power is, and the separation gap g1 may be greater than the diameter s1 of the wave w. Therefore, since the diameter r1 of the first through hole h1 through which the wave w of the wave source 110 passes is smaller than the diameter r2 of the second through hole h2 corresponding to the detector 120, the diameter s1 of the wave w of the water examination device 100 according to an embodiment of the present disclosure may be small, and thus, the range of the separation gap g1 may be designed to be increased.

Referring to FIG. 8, the first through hole h1 and the second through hole h2 of the fluid accommodation unit 160 may be formed at facing positions of the wall portion 162. In other words, the first through hole h1 and the second through hole h2 may be formed at positions facing each other based on a first axis Ax1 passing through the center of the bottom portion 161.

At this time, the first through hole h1 may be located in an upper portion of the wall portion 162 based on a half line HL passing through a half of the height of the wall portion 162, and the second through hole h2 may be located in a lower portion of the wall portion 162 based on the half line HL. Since the second through hole h2 is located at a lower level than the first through hole h1, external light, which may enter the open upper portion of the fluid accommodation unit 160, may be prevented from entering the detector 120. In other words, since the detector 120 of the water examination device 100 according to the present invention is located at a lower portion of the fluid accommodation unit 160, noises may be reduced and thus the detection accuracy may be increased.

Referring to FIG. 9, the fluid accommodation unit 160 has an open upper portion and may directly accommodate the fluid L, or may accommodate the fluid L using an external member containing the fluid L, for example, a cup 190. A user may pour the fluid L above the minimum acceptance line indicated on the cup and put the cup on the fluid accommodation unit 160 of the water examination device 100.

When the examination starts, the wave source 110 irradiates the wave w toward the fluid accommodation unit 160. In this regard, the central light passing through the center of the light path through which the wave w passes is irradiated to a position opposite to the wave source 110, and a part of the wave w may collide with materials in the fluid L and cause scattering.

When there is no foreign material, such as microbe M inside the fluid L, the scattering of the wave w may have regularity on average, and the change in the laser speckle may not be large over time. In an embodiment, when a foreign material such as microbe M exists inside the fluid L, a part of the wave w may collide with the foreign material, such as the microbe M, and may show a scattering pattern that is different from a case where the foreign material, such as the microbe M, does not exist. Due to the different scattering patterns of the wave w, the laser speckle changes over time, and the detector 120 may detect the laser speckle in time series to confirm the existence of microbes and estimate the concentration of microbes.

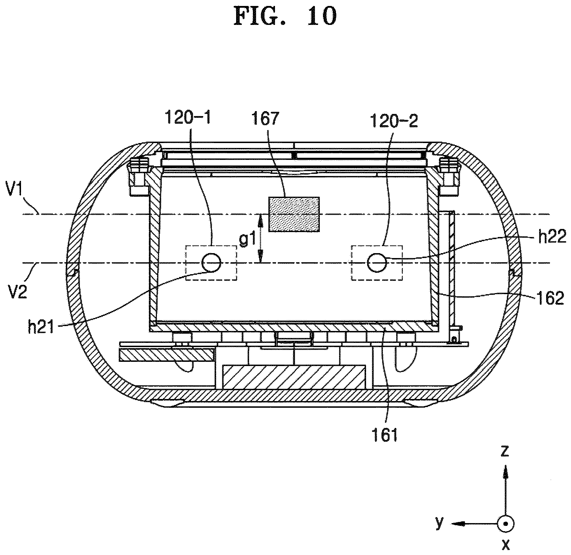

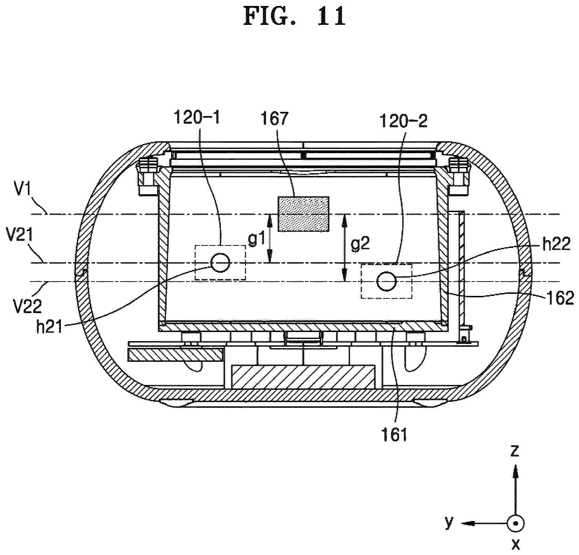

FIGS. 10 and 11 are diagrams provided to describe a water examination device 100 according to another embodiment of the present disclosure.

Referring to FIGS. 10 and 11, the water examination device 100 according to another embodiment of the present disclosure may include two or more detectors. The water examination device 100 includes two or more detectors, and a foreign material, such as a microbe M, may be detected by measuring a change in the laser speckle over time detected by each detector.

When two or more detectors are provided, the detectors may be disposed at different locations. As illustrated in FIG. 10, when a first detector 120-1 and a second detector 120-2 are included, the first detector 120-1 and the second detector 120-2 may be symmetrically disposed with respect to a certain area 167 of the fluid accommodation unit 160. However, the arrangement thereof is not limited thereto, and the first detector 120-1 may be disposed at a position facing the wave source 110, and the second detector 120-2 may be disposed at a position which is perpendicular to or forms a certain angle with respect to the direction in which the wave w is irradiated from the wave source 110. The first detector 120-1 and the second detector 120-2 may be positioned on the same line in the z direction, and the imaginary second plane V2 may pass through the first detector 120-1 and the second detector 120-2 at the same time.

In an embodiment, as illustrated in FIG. 11, when two or more detectors are provided, the first detector 120-1 and the second detector 120-2 may be disposed at different positions in the z direction. In other words, the first detector 120-1 and the second detector 120-2 may not be symmetrically disposed, and may be disposed at any position that enables the detection of a laser speckle.

Even when a foreign material, such as the microbe M, exists, the position thereof is not always fixed due to the nature of the fluid L. Accordingly, by having two or more detectors, the laser speckle may be detected in time series at each position, and the detection accuracy of the foreign material, such as the microbe M, may be improved.

In this regard, a 2-1 plane V21 passing through the first detector 120-1 and a 2-2 plane V22 passing through the second detector 120-2 may be parallel to the first plane V1 passing through the wave source 110. In this case, the first plane V1 and the 2-1 plane V21 have a first separation gap g1, and the first plane V1 and the 2-2 plane V22 passing through the second detector 120-2 have a second separation gap g2. A shorter distance from among the first separation gap g1 and the second separation gap g2 may be greater than at least the diameter s1 of the wave w. As such, the wave w irradiated from the wave source 110 may not be directly irradiated to the first detector 120-1 or the second detector 120-2.

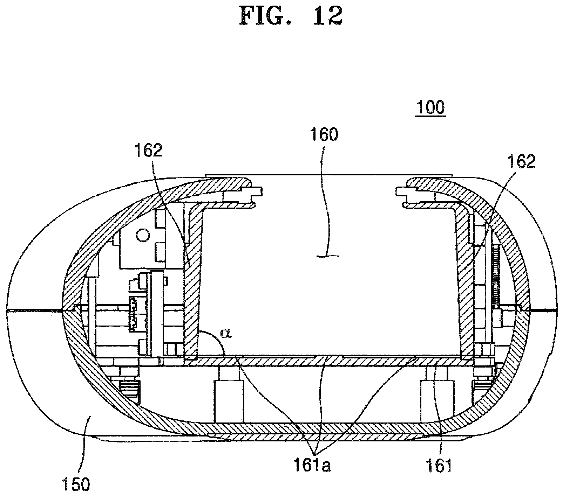

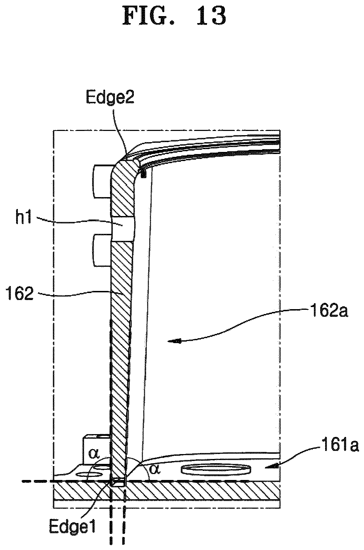

FIG. 12 is a cross-sectional view taken along line B-B' of FIG. 4, and FIG. 13 is a plan view of the water examination device of FIG. 4.

Referring to FIGS. 12 and 13, the water examination device 100 according to an embodiment of the present disclosure include three or more support portions 161a protruding from the bottom portion 161 so as to stably support the cup 190 which is accommodated in the fluid accommodation unit 160.

In detail, the cup 190 for containing the fluid has various shapes, and may not have a flat bottom surface. In this case, when the cup 190 is seated in the fluid accommodation unit 160, the cup 190 may not be stabilized, but finely shakes such that an error may occur in the measured value.

To address the above issue, according to an embodiment of the present disclosure, the three or more support portions 161a protrude from the bottom portion 161 and stably support the cup 190 accommodated in the fluid accommodation unit 160.

As described above, when three or more support portions 161a protrude from the bottom portion 161, the bottom portion 161 and the bottom surface of the cup 190 are in three-point (or more) contact with each other, not in the surface contact with each other. Through the three-point contact, fine shaking that may occur when the bottom surface of the cup 190 is not flat may be minimized, and an accuracy during repeated measurements may be improved.

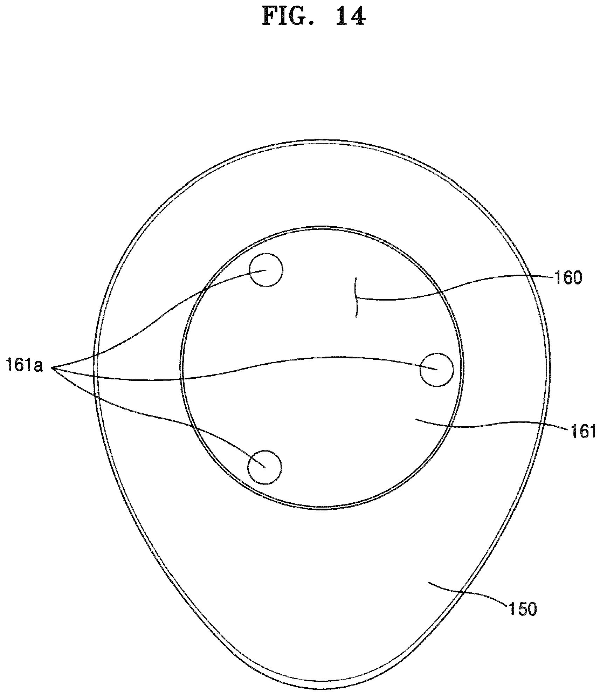

FIG. 14 is a diagram provided to explain the structure of the wall portion and the bottom portion 161 of the fluid accommodation unit.

Referring to FIG. 14, the wall portion 162 may be formed in a substantially vertical direction from the bottom portion 161 and has an annular shape so that the cup 190 may be accommodated therein. Here, in the water examination device 100 according to an embodiment of the present disclosure, an angle ($\alpha$) between the wall portion 162 and the bottom portion 161 is not a right angle, but is slightly inclined (that is, oblique).

In detail, the water examination device 100 according to an embodiment of the present disclosure has a structure, in which the portion where the fluid accommodation unit 160 is formed is opened to accommodate the cup 190. When the light is scattered due to the reflection of laser in the above structure in which one surface (of which at least a part) is opened, the scattered light is partially emitted out of the opened structure, and thus, there is a loss of the light intensity received by the detector 120 (see FIG. 2), which affects a result obtained through the light scattering analysis.

To address the above issue, according to one embodiment of the present disclosure, the angle ($\alpha$) formed by the wall portion 162 and the bottom portion 161 is not the right angle, but is slightly inclined. That is, the wall portion 162 is formed such that the angle ($\alpha$) formed by the wall portion 162 and the bottom portion 161 is about 85° to 88°, and thus, the laser reflected by the wall portion 162 proceeds toward a side opposite to the opened surface to reduce the loss of light intensity. Therefore, the light scattering effect may be improved.

In other words, the wall portion 162 may be formed to have a narrowed entrance in an upward direction (that is, in a +z direction), or may have a diameter that is gradually reduced upward. According to the present disclosure, the loss of light emitted from the wave source 110 is reduced, and the light scattering effect may be improved.

In an embodiment, the wall portion 162 may be formed such that an angle $\alpha$ between an inner surface 162-1 of the wall portion 162 and an inner surface 161-1 of the bottom portion 161 may be about 85° to 88°, and an angle between an outer surface of the wall portion 162 and the inner surface 161-1 of the bottom portion 161 may be 90°. In other words, the wall portion 162 may have a first edge Edge1 adjacent to the bottom portion 161 and a second edge Edge2 adjacent to the open portion of the wall portion 162, wherein the cross-sectional thickness of the second edge Edge2 may be greater than the cross-sectional thickness of the first edge Edge1. As such, the outer diameter of the wall portion 162 may be the same in the z direction, and the inner diameter of the wall portion 162 may be gradually decreased upward (that is, in the +z direction).

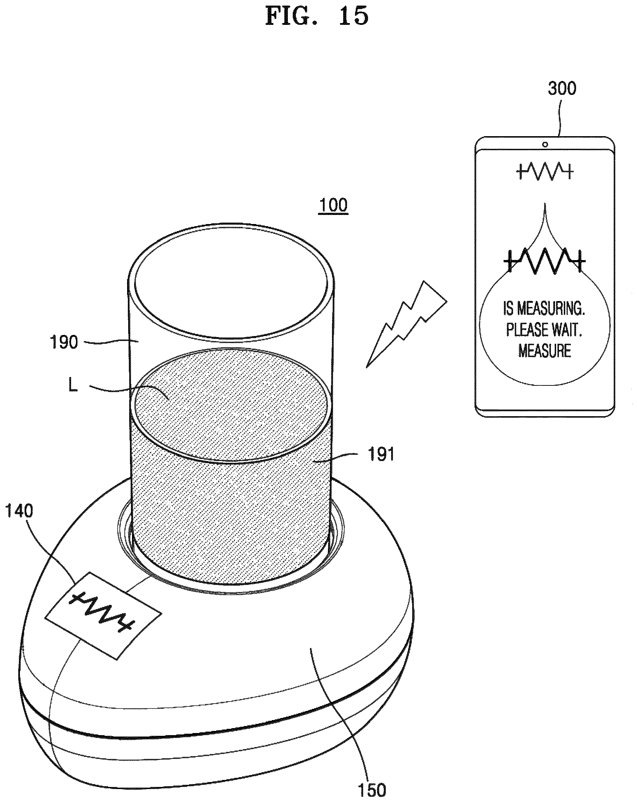

FIG. 15 is a diagram illustrating the use of a water examination device.

Referring to FIG. 15, the user may place the cup 190 containing the fluid L on the fluid accommodation unit 160 of the water examination device 100 and then apply an input signal to start the examination through a user interface. The user interface may be included in the water examination device 100, and may be provided to a user terminal 300 as illustrated the diagram to transmit an input signal to the water examination device 100 through communication.

In addition, the cup 190 may include an opaque portion 191. In detail, when the cup 190 is formed to be entirely transparent, the laser may be exposed to outside, and the user may feel the glaring effect. Also, there is a need to indicate an amount of minimum fluid that is necessary for examining the quality of fluid in the cup 190 by using the water examination device 100.

In order to address the above issue, the opaque portion 191 is formed in at least a part of the cup 190, in particular, to a certain height at a side lower portion of the cup 190, such that the user may identify the minimum flow amount that is necessary for examining the fluid quality, and at the same time, the user may not directly see the laser with naked eyes.

Meanwhile, when the water quality examination of the fluid L is completed, the water examination device 100 may notify the user of the result through the alarm unit 140. The alarm unit 140 may notify that there are microbes in the fluid by using any one of sound and light. The alarm unit 140 may include an illumination unit such as an LED generating an alarm signal via light and a speaker (not shown) generating an alarm signal via sound, and the light and the sound may be simultaneously generated. However, when microbes do not exist in the fluid, the alarm unit 140 may externally show the signal indicating that fluid L is safe.

For example, when the concentration of microbes in the fluid is below a preset reference value, the alarm unit 140 may emit green light, and when the concentration of microbes in the fluid is greater than a preset reference value and is within the present range, the alarm unit 140 may emit orange light. When the concentration of microbes in the fluid exceeds the preset range, the alarm unit 140 may emit red light, informing the user of the danger.

Meanwhile, the water examination device 100 may transmit result data to the user terminal 300 through communication, and may display the presence or absence of microbes and the microbes concentration information through a display of the user terminal 300.

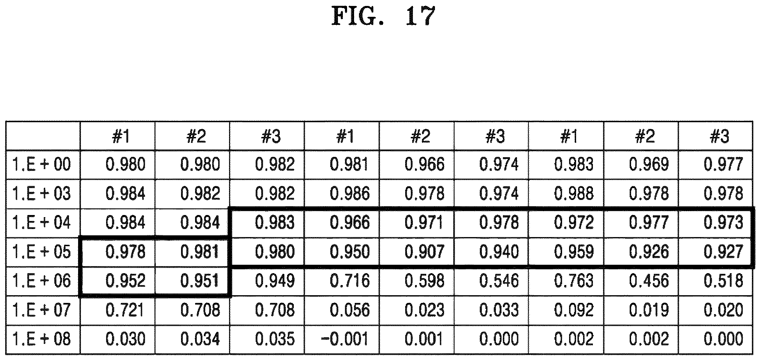

FIG. 16 is a graph of a temporal correlation coefficient according to a separation gap between a wave source and a detector, obtained by using the water examination device according to an embodiment of the present disclosure, and FIG. 17 is a table listing temporal correlation coefficients according to concentrations according to the separation gap between the wave source and the detector.

Referring to FIG. 16, C1 represents a reference graph when the separation gap is a first value, C2 represents a graph when the separation gap is the first value and the inner surface of the fluid accommodation unit 160 is formed of a light-absorbing material, and C3 represents a graph when the separation gap is a half of the first value and the inner surface of the fluid accommodation unit 160 is formed of a light-absorbing material.

Referring to FIG. 16, when the separation gap between the wave source 110 and the detector 120 is the same, and the inner surface of the fluid accommodation unit 160 is formed of a light-absorbing material (C2), the change rate of the temporal correlation coefficient according to the concentration of microbes is greater than that of C1. In addition, when the separation gap between the wave source 110 and the detector 120 is reduced by half (C3, the light-absorbing material is the same), it can be confirmed that the change rate of the temporal correlation coefficient according to the concentration of microbes is greater than that of C2.

When the concentration of microbes is different and the change in the temporal correlation coefficient is not large, it becomes difficult to distinguish even when the concentration of microbes is different. Therefore, the detection ability of the water examination device 100 is improved as the rate of change of the temporal correlation coefficient is increased. It can be seen that the rate of change of the temporal correlation coefficient increases as the separation gap between the wave source 110 and the detector 120 decreases.

In an embodiment, referring to FIG. 17, Comparative Example is a case where the separation gap is a first value, and Example 1 is a case where the inner surface of the fluid accommodation unit is formed of a light-absorbing material and the separation gap between the wave source 110 and the detector 120 is a half of the first value, and Example 2 is a case where the inner surface of the fluid accommodation unit is formed of a light-absorbing material and the separation gap between the wave source 110 and the detector 120 is one fourth of the first value.

In the case of Comparative Example, the temporal correlation coefficient changes when the concentration of microbes is 10^5 cfu/ml to 10^6 cfu/ml, whereas in the case of Example 1 or Example 2, the temporal correlation coefficient changes when the concentration of microbes is 10^3 cfu/ml to 10^4 cfu/ml. In other words, when the separation gap between the wave source 110 and the detector 120 is short, it can be confirmed that microbes with a lower concentration can be detected.

Meanwhile, although not illustrated, as the plane distance d1 (see FIG. 5) between the wave source 110 and the detector 120 is longer, the change rate of the temporal correlation coefficient of the laser speckle may be greater. The longer the plane distance d1 between the wave source 110 and the detector 120 is, the longer the path length of the wave w in the fluid L is, so that the light scattering of the laser speckle can be increased, thereby enhancing the detection power.

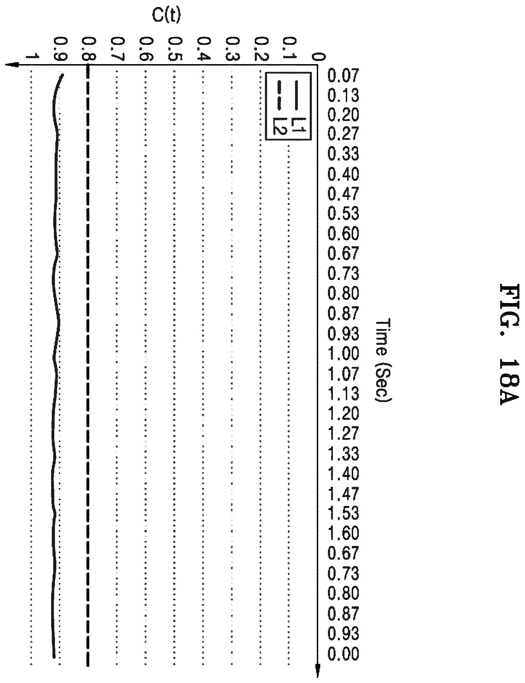

FIGS. 18A to 18C are graphs showing a temporal correlation coefficient according to a bacterial concentration in a fluid in a water examination device according to an embodiment of the present disclosure. FIGS. 18A to 18C show changes in the temporal correlation coefficient according to a concentration of microbes, when microbes are artificially injected into the fluid contained in the cup 190.

In the graphs of FIGS. 18A to 18C, an x-axis denotes an axis regarding time (t) and y-axis denotes an axis regarding the temporal correlation coefficient C(t). Here, a dashed line L2 denotes a reference value of the temporal correlation coefficient of the laser speckle set in advance in the detector 120. Also, a solid line L1 denotes measurement data of the temporal correlation coefficient of the laser speckle obtained over time through the detector 120.

The solid line L1 of FIG. 18A denotes the temporal correlation coefficient of the laser speckle obtained by the detector 120, when the microbes are not injected in the fluid. Referring to FIG. 9A, when there are no microbes in the fluid, there is no change in the laser speckle generated due to the scattering in the fluid, and thus, the temporal correlation coefficient is nearly consistent over time and may not exceed the reference value L1 set in advance.

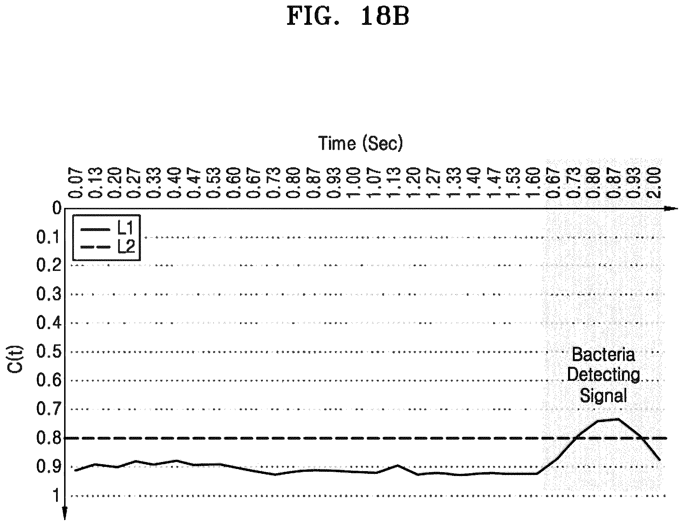

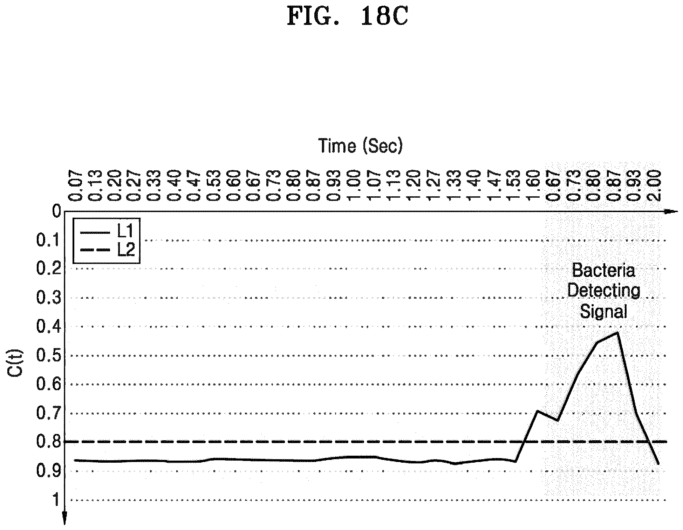

The solid line L1 of FIG. 18B denotes the temporal correlation coefficient of the laser speckle obtained by the detector 120 when 4 ml of microbes with a concentration of 10^0 cfu/ml is injected in the fluid. The solid line L1 of FIG. 18C denotes the temporal correlation coefficient of the laser speckle obtained by the detector 120 when 4 ml of microbes with a concentration of 10^1 cfu/ml is injected in the fluid.

Referring to FIGS. 18B and 18C, when there are microbes in the fluid, the laser speckles generated due to the scattering in the fluid change over time, and thus, the temporal correlation coefficient changes at the time when the microbes are sensed. In FIGS. 18B and 18C, a shaded region (bacteria detecting signal) denotes the change in the temporal correlation coefficient at the time of detecting the microbes, and it can be seen that as the concentration of the microbes is increased, a peak of the temporal correlation coefficient is increased. In addition, regarding the shaded region of FIGS. 18B and 18C, the detector 120 may determine that there are the microbes when the temporal correlation coefficient L1 of the laser speckle exceeds the dashed line L2, that is, the reference value set in advance. Here, when there are microbes, a measurement time taken for the detector 120 to sense the microbes may be a section from a time when the temporal correlation coefficient rapidly changes and to a point where the temporal correlation coefficient meets the dashed line L2, that is, the reference value, and with reference to FIGS. 18B and 18C, the measurement time may be about 0.2 second or less.

As such, the water examination device according to the embodiments of the present disclosure may sense the microbes that are the foreign material in the fluid within a very short time period, e.g., 0.2 sec. or less, that is, in real-time. Also, the water examination device according to the embodiments of the present disclosure may estimate the concentration of the microbes by using the change ratio of the temporal correlation coefficient or the peak value. Also, the water examination device may detect the microbes even when the concentration of the microbes is low.

As described above, the water examination device according to the embodiments of the present disclosure may estimate whether there are microbes in the fluid or the concentration of the microbes at low costs, by using the change in the temporal correlation of the laser speckle.

Hereinafter, the method of detecting the microbes in the water examination device according to another embodiment of the present disclosure will be described below. The method of detecting the microbes in the water examination device according to another embodiment of the present disclosure may detect whether there are the microbes in the fluid or the concentration of the microbes by using a spatial correlation, instead of the temporal correlation. Hereinafter, the above method will be described below in more detail.

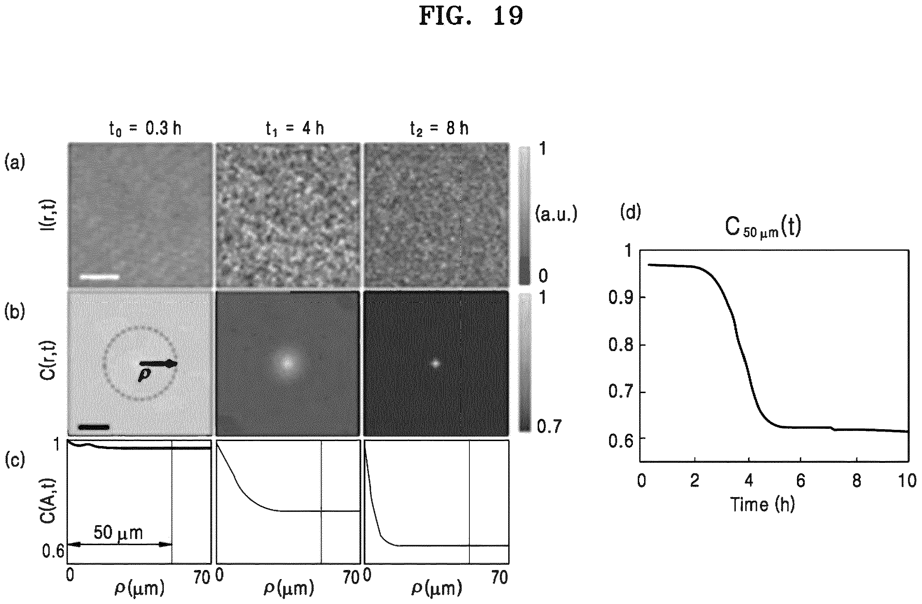

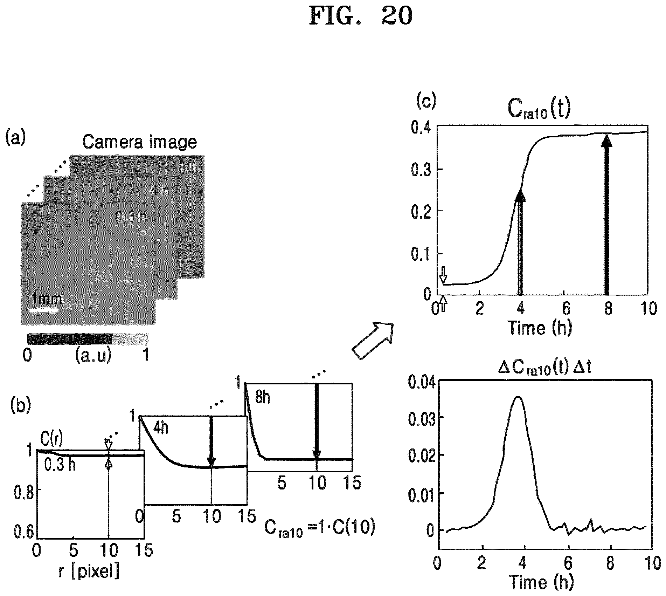

FIGS. 19 and 20 are diagrams for describing a principle of detecting microbes in a water examination device according to another embodiment of the present disclosure.

Referring to FIGS. 19 and 20, the controller 130 receives an optical image measured in the time-serial manner from the controller 120, and may determine information about concentration of microbes in the sample from the optical image.

The controller 130 may obtain a spatial correlation of an interference pattern. Here, the spatial correlation expressed by Equation below may indicate how a brightness of an arbitrary pixel and a brightness of a pixel spaced apart from that pixel by a distance r are similar to each other on an image measured at a time t, in a number within a certain range (see (b) of FIG. 19). The certain range may be a range from -1 to 1. That is, the spatial correlation indicates a correlation degree between an arbitrary pixel and another pixel, and 1 denotes a positive correlation, -1 denotes a negative correlation, and 0 denotes no correlation. In particular, because the illuminance is emitted evenly before forming the interference pattern, the spatial correlation of a sample image indicates a positive correlation close to 1. However, after forming the interference pattern, the value of correlation may decrease in the direction toward 0.

The detector 120 may define a brightness measured at time t in a pixel at a location r'=(x,y) as I(r',t), and may define a brightness of a pixel spaced by a distance r as I(r'+r, t). The spatial correlation may be expressed by Inequality 5 below by using the above.

## [Equation 5]

$$C(r,t) = \frac{1}{C_0(t)} \iint I(r'+r,t)I(r',t)dr'$$

C0(t) is used to set the range of Equation 5 between -1 to 1. When the brightness I(r',t) measured at time t in an arbitrary pixel and the brightness I(r'+r,t) of the pixel spaced by a distance r are equal to each other, the spatial correlation is 1, and the spatial correlation is less than 1 when the above brightness are not equal to each other.

In an embodiment, the spatial correlation as described above may be only represented as a function for time. To this end, the controller 130 may calculate an average of the spatial correlation with respect to a pixel having the same size of the distance r from an arbitrary pixel by using Equation 6 below (see (b) of FIG. 19).

## [Equation 6]

$$C(\rho,t) = \frac{1}{2\pi} \int_0^{2\pi} C(r,t)d\theta$$

In an embodiment, the controller 130 may substitute a distance set in advance in Equation 6 above to represent a function for the time, and may identify a degree of forming the interference pattern as a value within a certain range, e.g., 0 to 1, by using the function (see (d) of FIG. 19).

The controller 130 may distinguish foreign matters from microbes in the sample through a change in the pattern of the sample image over time. In case of the foreign matters, there is no change in the image over time, but in case of the microbes, there is a change in the shape, the size, etc. in the image over time. Thus, the water examination device 100 may distinguish the foreign matters from the microbes.

In addition, the controller 130 may determine information on the concentration of microbes by using the spatial correlation. The spatial correlation may be obtained by generating two identical overlaid images by using one image, shifting one of the two images as much as a distance set in advance in one direction, and analyzing how two adjacent pixels in the shifted image and non-shifted image are similar to each other. Here, the spatial correlation becomes a criterion representing how the image is uniform. When an interference pattern is generated due to a colony, similarity between two adjacent pixels degrades due to small interference patterns, and a value of the spatial correlation also degrades.

The spatial correlation coefficient varies depending on the shifted distance r (see (b) of FIG. 19), and within a certain distance range, as the shifted distance r increases, the value of the spatial correlation coefficient decreases, and when exceeding the certain distance range, the value of the spatial correlation coefficient is nearly consistent. Therefore, in order to obtain more significant spatial correlation, the controller 130 may obtain the spatial correlation by shifting the image by a certain distance set in advance or greater. Here, the certain distance r set in advance is dependent upon a speckle size, and the controller 130 may obtain the spatial correlation by shifting the image as much as a pixel size greater than the speckle size when expressed in units of pixel. For example, the certain distance set in advance may be at least three-pixel distance or greater.

In addition, the controller 130 obtains the temporal correlation of the interference pattern in the sample image, as well as the spatial correlation as above, and may detect the microbes based on the obtained temporal correlation. The controller 130 may calculate the temporal correlation coefficient between images by using information about images of the interference pattern measured in the time-serial manner, and may detect a microbe colony in the sample based on the temporal correlation coefficient.

The controller 130 may detect the microbes through the analysis about decreasing of the calculated temporal correlation coefficient to a reference value set in advance or less.

Hereinafter, a water monitoring method according to an embodiment of the present disclosure will be described below. FIG. 21 is a flowchart illustrating a water monitoring method according to an embodiment of the present disclosure.

Referring to FIG. 21, the water monitoring method according to an embodiment of the present disclosure includes: identifying, by the water examination device 100, whether the calibration is made (S110); performing the calibration in the water examination device 100 (S120); detecting water quality data by the water examination device 100 in which the calibration is performed (S130); transferring the detected water quality data from the water examination device 100 to the server 200 (S140); collecting the detected water quality data by the server 200 (S150); generating, by the server 200, water quality processed data by using position and/or time information (S160); transferring the generated water quality processed data from the server 200 to the user terminal 300 (S170); and displaying the water quality processed data on the user terminal 300 (S180).

First, the water examination device 100 identifies whether the calibration is performed (S110). That is, the controller 130 of the water examination device 100 identifies whether the calibration is performed in the water examination device 100, based on whether there is a calibration value stored in advance (for example, a shutter speed value, a voltage at the wave source, etc.).

Next, when the calibration is not performed in the water examination device 100, the water examination device 100 performs a calibration (S120). That is, the calibration unit 180 controls the water examination device 100 in order for the intensity of light measured by the detector 120 to be in a certain range set in advance. This will be described in detail later with reference to FIG. 13.

Next, water quality data is detected from the water examination device 100 in which the calibration is performed (S130). In detail, the detector 120 may detect the laser speckles that are generated when the irradiated waves are multiple scattered in the fluid L, at every preset time. The detector 120 may detect the laser speckle at a first time at least, and may detect the laser speckle at a second time, and then, may provide the controller 130. The first time and the second time are just examples selected for convenience of description, and the detector 120 may detect laser speckles at a plurality of times more than the first and second times.

Next, the detected water quality data is transferred from the water examination device 100 to the server 200 via the network 400 (see FIG. 1) (S140). The water quality data is collected by the server 200 (S150).

Next, the server 200 generates water quality processed data by using position and/or time information (S160). In detail, the server 200 may collect water quality data examined by each water examination device 100, processes the water quality data, and provides the water quality data processed as above to the user terminals 300. Here, the water quality data processed by the server 200 and provided to the user terminal 300 may include a water quality level measured by the corresponding user terminal 300 in comparison with a total water quality average, a water quality level in a region where the corresponding user terminal 300 is located in comparison with the total water quality average, a deviation in water quality according to the region, a variation in the water quality over time, etc.

Next, the generated water quality processed data is transferred from the server 200 to the user terminal 300 via the network 400 (see FIG. 1) (S170). In addition, the water quality processed data is displayed on the user terminal 300 (S180).

Hereinafter, the process of performing the calibration in the water examination device 100 will be described in detail below.

FIG. 22 is a flowchart illustrating operation S120 of FIG. 21 in more detail.

Referring to FIG. 22. the method of detecting microbes in the water examination device according to another embodiment of the present disclosure includes: measuring, by the detector 120, the intensity of light that is irradiated from the wave source 110 and passes through a fluid L (S121); determining whether the light intensity measured by the detector 120 is equal to or greater than a first reference value (S122); when the light intensity measured by the detector 120 is not equal to or greater than the first reference value, controlling, by the calibration unit 180, the shutter speed of the shutter 122 to be decreased (S123); when the light intensity measured by the detector 120 is equal to or greater than the first reference value, determining whether the light intensity measured by the detector 120 is equal to or less than a second reference value (S124); when the light intensity measured by the detector 120 is not equal to or less than the second reference value, controlling, by the calibration unit 180, the shutter speed of the shutter 122 to be increased (S125); when the light intensity measured by the detector 120 is equal to or less than the second reference value, storing the current shutter speed (S126); and obtaining, by the controller 130, a temporal correlation of the laser speckle, by using the image detected by the detector 120 (S127).

This will be described below in more detail.

First, the intensity of light irradiated from the wave source 110 and passed through the fluid L is measured by the detector 120 (S121). Here, the process of measuring the light irradiated from the wave source 110 in the detector 120 is described in detail above, and detailed descriptions thereof are omitted.

Next, it is determined whether the light intensity measured by the detector 120 is equal to or greater than the first reference value (S122). Here, a calibration reference value may be determined from an average of intensity values of respective pixels in an image detected by the detector 120.

In detail, in case of an 8-bits image sensor, an intensity of each pixel has a value between 0 to 255, and an intermediate value, that is, 128, may be set as a reference value. In addition, based on various experimental data, an appropriate light intensity range may be set as $\pm 5$ from the reference value. In addition, a minimum value of the appropriate light intensity range is set as a first reference value and a maximum value of the appropriate light intensity range may be set as a second reference value.

For example, when the average value of the intensities ranges from 123 to 133, it may be set as an appropriate light intensity. (Here, the first reference value may be 123 and the second reference value may be 133.) In addition, an average of the intensity values of the pixels in the image that is irradiated from the wave source 110 and detected by the detector 120 after passing through the fluid L is measured, and when the average of the intensity values is less than the first reference value, e.g., 123, the calibration unit 180 determines that the light intensity is not sufficient and controls the shutter speed of the shutter 122 to be decreased in order to increase the intensity of light incident to the sensor unit 121 (S123).

When the light intensity measured by the detector 120 is equal to or greater than the first reference value, it is determined whether the light intensity measured by the detector 120 is equal to or less than the second reference value (S124).

As a result of determination, when the average of the intensity values of the pixels in the detected image is greater than the second reference value, e.g., 133, the calibration unit 180 determines that the light intensity is excessive and controls the shutter speed of the shutter 122 to be increased in order to decrease the intensity of the light incident to the sensor unit 121 (S125).

In addition, when the light intensity measured by the detector 120 is equal to or less than the second reference value, the current shutter speed is stored (S126).

Last, the controller 130 obtains the temporal correlation of the laser speckle by using the image detected by the detector 120 (S127). Here, the process of obtaining the temporal correlation of the laser speckle is described above in detail, and detailed descriptions thereof are omitted.

As described above, after calibrating the intensity of light detected by the detector 120 within a certain range by using the calibration unit 180, the controller 130 may obtain the temporal correlation of the laser speckle. Thus, even when the performance of the wave source 110 degrades, deterioration such as mosses grown in the detector 120 occurs, or there is a deviation in container containing the fluid sample, an error in obtaining the temporal correlation of the laser speckle may be minimized.

While the present disclosure has been particularly shown and described with reference to preferred embodiments thereof, it will be understood by those skilled in the art that various changes in form and details may be made therein without departing from the spirit and scope of the present disclosure as defined by the appended claims. The preferred embodiments should be considered in descriptive sense only and not for purposes of limitation. Therefore, the scope of the present disclosure is defined not by the detailed description of the disclosure but by the appended claims, and all differences within the scope will be construed as being included in the present disclosure.

Industrial Applicability

According to an embodiment of the present disclosure, a water examination device is provided. In addition, embodiments of the present disclosure may be applied to impurity or microbes detection devices that are used industrially.

**Claims**

1. A water examination device comprising:

   a main body;
   a fluid accommodation unit formed in the main body;
   a wave source for irradiating waves toward the fluid accommodation unit;
   a detector for detecting a laser speckle in time series, the laser speckle being generated due to multiple scattering of the waves in the fluid; and
   a controller for estimating existence of a foreign material in the fluid in real-time by using the detected laser speckle, wherein
   an imaginary first plane passing through the wave source is parallel to an imaginary second plane passing through the detector.

2. The water examination device of claim 1, wherein

   the imaginary first plane passes through an irradiation plane on which the wave source irradiates the wave, and
   the imaginary second plane passes through a detection plane of the detector.

3. The water examination device of claim 1, wherein
   a distance between the imaginary first surface and the imaginary second surface is larger than a diameter of the wave irradiated from the wave source.

4. The water examination device of claim 1, wherein
   the fluid accommodation unit includes:

   a bottom portion formed in the main body; and

a wall portion formed to have a certain angle with respect to the bottom portion.

5. The water examination device of claim 4, wherein
   a certain area of the inner surface of the wall portion to which the wave is irradiated, is formed of a light-absorbing material.

6. The water examination device of claim 5, wherein the certain area to which the wave is irradiated, is larger than the diameter of the wave.

7. The water examination device of claim 5, wherein the inner surface of the wall portion is entirely formed of a light-absorbing material.

8. The water examination device of claim 7, wherein the bottom portion has an inner surface which is entirely formed of a light-absorbing material.

9. The water examination device of claim 1, wherein the fluid accommodation unit has a first through hole corresponding to the position of the wave source and through which the wave passes, and a second through hole corresponding to the position of the detector.

10. The water examination device of claim 9, wherein the imaginary first plane passes through the first through hole, and does not pass through the second through hole.

11. The water examination device of claim 9, wherein a diameter of the first through hole is smaller than a diameter of the second through hole.

12. The water examination device of claim 1, wherein
    the controller obtains a temporal correlation of the laser speckle which is detected by using the detected laser speckle, and estimates the presence or absence of microbes in the fluid in real-time based on the obtained temporal correlation.

13. The water examination device of claim 12, wherein the temporal correlation includes a difference between first image information of the laser speckle detected at a first time and second image information of the laser speckle detected at a second time that is different from the first time.

14. The water examination device of claim 13, wherein each of the first image information and the second image information comprise at least one of pattern information of the laser speckle and intensity information of the wave.

15. The water examination device of claim 1, wherein the controller obtains a spatial correlation of an interference pattern in an optical image detected by the detector and determines existence of a foreign material in the fluid based on a change in the spatial correlation of the interference pattern over time.

# FIG. 1

# FIG. 2

EP 4 400 831 A1

# FIG. 3

Static speckle fields

laser

Dynamic speckle fields

laser

Culture dish

Living
Microbes

Multiple light scattering, static

Multiple light scattering, dynamic

# FIG. 4

FIG. 5

FIG. 6

# FIG. 7

# FIG. 8

# FIG. 9

FIG. 10

FIG. 11

# FIG. 12

# FIG. 13

# FIG. 14

# FIG. 15

# FIG. 16

FIG. 17

|  | #1 | #2 | #3 | #1 | #2 | #3 | #1 | #2 | #3 |
|---|---|---|---|---|---|---|---|---|---|
| 1.E + 00 | 0.980 | 0.980 | 0.982 | 0.981 | 0.966 | 0.974 | 0.983 | 0.969 | 0.977 |
| 1.E + 03 | 0.984 | 0.982 | 0.982 | 0.986 | 0.978 | 0.974 | 0.988 | 0.978 | 0.978 |
| 1.E + 04 | 0.984 | 0.984 | 0.983 | 0.966 | 0.971 | 0.978 | 0.972 | 0.977 | 0.973 |
| 1.E + 05 | 0.978 | 0.981 | 0.980 | 0.950 | 0.907 | 0.940 | 0.959 | 0.926 | 0.927 |
| 1.E + 06 | 0.952 | 0.951 | 0.949 | 0.716 | 0.598 | 0.546 | 0.763 | 0.456 | 0.518 |
| 1.E + 07 | 0.721 | 0.708 | 0.708 | 0.056 | 0.023 | 0.033 | 0.092 | 0.019 | 0.020 |
| 1.E + 08 | 0.030 | 0.034 | 0.035 | −0.001 | 0.001 | 0.000 | 0.002 | 0.002 | 0.000 |

EP 4 400 831 A1

# FIG. 18A

# FIG. 18B

# FIG. 18C

FIG. 19

EP 4 400 831 A1

# FIG. 20

# FIG. 21

WATER EXAMINATION DEVICE ⌐100

USER TERMINAL ⌐300

SERVER ⌐200

IDENTIFY WHETHER CALIBRATION IS MADE — S110

PERFORM CALIBRATION — S120

DETECT WATER QUALITY DATA — S130

TRANSFER DETECTED WATER QUALITY DATA (S140)

COLLECT DETECTED WATER QUALITY DATA — S150

GENERATE WATER QUALITY PROCESSED DATA BY USING POSITION INFORMATION AND TIME INFORMATION — S160

TRANSFER WATER QUALITY PROCESSED DATA (S170)

DISPLAY WATER QUALITY PROCESSED DATA ON USER TERMINAL — S180

# FIG. 22

START

MEASURE INTENSITY OF LIGHT — S121

S122
INTENSITY OF LIGHT ≥ FIRST REFERENCE VALUE? — NO → DECREASE SHUTTER SPEED — S123

YES

S124
INTENSITY OF LIGHT ≥ SECOND REFERENCE VALUE? — NO → INCREASE SHUTTER SPEED — S125

YES

STORE SHUTTER SPEED — S126

OBTAIN TEMPORAL CORRELATION — S127

END

## INTERNATIONAL SEARCH REPORT

| International application No. |
|---|
| **PCT/KR2022/013103** |

**A.   CLASSIFICATION OF SUBJECT MATTER**

**G01N 21/49**(2006.01)i; **G01N 21/47**(2006.01)i; **G01N 21/45**(2006.01)i; **G01N 33/18**(2006.01)i; **G01N 21/51**(2006.01)i

According to International Patent Classification (IPC) or to both national classification and IPC

**B.   FIELDS SEARCHED**

Minimum documentation searched (classification system followed by classification symbols)

G01N 21/49(2006.01); G01N 15/10(2006.01); G01N 15/14(2006.01); G01N 21/47(2006.01); G01N 21/53(2006.01); G01N 33/02(2006.01); G01N 35/08(2006.01); G01N 37/00(2006.01); G03B 15/00(2006.01); H04N 5/225(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 수질(water quality), 혼돈파(chaotic wave), 레이저 스펙클(laser speckle), 이물질 (foreign matter)

**C.   DOCUMENTS CONSIDERED TO BE RELEVANT**

| Category* | Citation of document, with indication, where appropriate, of the relevant passages | Relevant to claim No. |
|---|---|---|
| X<br><br>Y | KR 10-2285089 B1 (THE WAVE TALK, INC.) 04 August 2021 (2021-08-04)<br>     See paragraphs [0022]-[0085] and figures 1-3 and 6. | 1-4,9-15<br><br>5-8 |
| Y | KR 10-1270773 B1 (KOREA INSTITUTE OF OCEAN SCIENCE & TECHNOLOGY) 03 June 2013 (2013-06-03)<br>     See paragraphs [0041]-[0045] and figures 1-4. | 5-8 |
| A | KR 10-1686766 B1 (KOREA ADVANCED INSTITUTE OF SCIENCE AND TECHNOLOGY) 15 December 2016 (2016-12-15)<br>     See paragraphs [0034] and [0035]. | 1-15 |
| A | US 2019-0107487 A1 (WYATT TECHNOLOGY CORPORATION) 11 April 2019 (2019-04-11)<br>     See paragraphs [0038]-[0045] and figures 4-7. | 1-15 |

☑ Further documents are listed in the continuation of Box C.          ☑ See patent family annex.

| | | |
|---|---|---|
| *     Special categories of cited documents: | "T" | later document published after the international filing date or priority date and not in conflict with the application but cited to understand the principle or theory underlying the invention |
| "A"   document defining the general state of the art which is not considered to be of particular relevance | | |
| "D"   document cited by the applicant in the international application | "X" | document of particular relevance; the claimed invention cannot be considered novel or cannot be considered to involve an inventive step when the document is taken alone |
| "E"   earlier application or patent but published on or after the international filing date | | |
| "L"   document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O"   document referring to an oral disclosure, use, exhibition or other means | "&" | document member of the same patent family |
| "P"   document published prior to the international filing date but later than the priority date claimed | | |

| Date of the actual completion of the international search | Date of mailing of the international search report |
|---|---|
| **23 December 2022** | **23 December 2022** |

| Name and mailing address of the ISA/KR | Authorized officer |
|---|---|
| **Korean Intellectual Property Office**<br>**Government Complex-Daejeon Building 4, 189 Cheongsa-**<br>**ro, Seo-gu, Daejeon 35208** | |
| Facsimile No. **+82-42-481-8578** | Telephone No. |

Form PCT/ISA/210 (second sheet) (July 2022)

<table>
<tr><td colspan="2" align="center">**INTERNATIONAL SEARCH REPORT**</td><td>International application No.<br>**PCT/KR2022/013103**</td></tr>
</table>

**C.    DOCUMENTS CONSIDERED TO BE RELEVANT**

| Category* | Citation of document, with indication, where appropriate, of the relevant passages | Relevant to claim No. |
|---|---|---|
| A | JP 2019-508687 A (SCANDINAVIAN MICRO BIODEVICES APS) 28 March 2019 (2019-03-28)<br>See claims 1-13. | 1-15 |

Form PCT/ISA/210 (second sheet) (July 2022)

**INTERNATIONAL SEARCH REPORT**
Information on patent family members

International application No.

**PCT/KR2022/013103**

| Patent document cited in search report | | | Publication date (day/month/year) | Patent family member(s) | | | Publication date (day/month/year) |
|---|---|---|---|---|---|---|---|
| KR | 10-2285089 | B1 | 04 August 2021 | US | 2021-0270735 | A1 | 02 September 2021 |
| KR | 10-1270773 | B1 | 03 June 2013 | None | | | |
| KR | 10-1686766 | B1 | 15 December 2016 | CN | 108474740 | A | 31 August 2018 |
| | | | | CN | 108474740 | B | 02 March 2021 |
| | | | | CN | 109313139 | A | 05 February 2019 |
| | | | | CN | 109313139 | B | 05 November 2021 |
| | | | | CN | 113063755 | A | 02 July 2021 |
| | | | | EP | 3171160 | A1 | 24 May 2017 |
| | | | | EP | 3171160 | B1 | 07 July 2021 |
| | | | | EP | 3379234 | A1 | 26 September 2018 |
| | | | | EP | 3467483 | A1 | 10 April 2019 |
| | | | | JP | 2018-536856 | A | 13 December 2018 |
| | | | | JP | 2019-518973 | A | 04 July 2019 |
| | | | | JP | 2022-106721 | A | 20 July 2022 |
| | | | | JP | 6851468 | B2 | 31 March 2021 |
| | | | | JP | 7058837 | B2 | 25 April 2022 |
| | | | | KR | 10-1959023 | B1 | 18 March 2019 |
| | | | | KR | 10-1971272 | B1 | 27 August 2019 |
| | | | | KR | 10-2017-0057827 | A | 25 May 2017 |
| | | | | KR | 10-2017-0058251 | A | 26 May 2017 |
| | | | | KR | 10-2017-0136989 | A | 12 December 2017 |
| | | | | KR | 10-2018-0010589 | A | 31 January 2018 |
| | | | | KR | 10-2018-0040364 | A | 20 April 2018 |
| | | | | KR | 10-2018-0040365 | A | 20 April 2018 |
| | | | | KR | 10-2018-0047853 | A | 10 May 2018 |
| | | | | KR | 10-2018-0055301 | A | 25 May 2018 |
| | | | | KR | 10-2018895 | B1 | 06 September 2019 |
| | | | | KR | 10-2055310 | B1 | 13 December 2019 |
| | | | | US | 10001467 | B2 | 19 June 2018 |
| | | | | US | 10551293 | B2 | 04 February 2020 |
| | | | | US | 10852246 | B2 | 01 December 2020 |
| | | | | US | 10914665 | B2 | 09 February 2021 |
| | | | | US | 11262287 | B2 | 01 March 2022 |
| | | | | US | 2017-0138923 | A1 | 18 May 2017 |
| | | | | US | 2018-0372608 | A1 | 27 December 2018 |
| | | | | US | 2019-0212276 | A1 | 11 July 2019 |
| | | | | US | 2020-0116618 | A1 | 16 April 2020 |
| | | | | US | 2021-0231550 | A1 | 29 July 2021 |
| | | | | WO | 2017-086719 | A1 | 26 May 2017 |
| | | | | WO | 2017-209544 | A1 | 07 December 2017 |
| US | 2019-0107487 | A1 | 11 April 2019 | US | 10466173 | B2 | 05 November 2019 |
| | | | | US | 10895531 | B2 | 19 January 2021 |
| | | | | US | 2020-0072747 | A1 | 05 March 2020 |
| | | | | US | 2021-0318237 | A1 | 14 October 2021 |
| JP | 2019-508687 | A | 28 March 2019 | AU | 2017-214175 | A1 | 19 July 2018 |
| | | | | AU | 2017-214175 | B2 | 23 September 2021 |
| | | | | BR | 112018015523 | A2 | 26 December 2018 |
| | | | | CA | 3013085 | A1 | 10 August 2017 |
| | | | | CN | 108698046 | A | 23 October 2018 |
| | | | | CN | 108698046 | B | 22 October 2021 |

Form PCT/ISA/210 (patent family annex) (July 2022)

**INTERNATIONAL SEARCH REPORT**
Information on patent family members

International application No.

**PCT/KR2022/013103**

| Patent document cited in search report | Publication date (day/month/year) | Patent family member(s) | | Publication date (day/month/year) |
|---|---|---|---|---|
| | | DK | 3411151 T3 | 26 July 2021 |
| | | EP | 3411151 A1 | 12 December 2018 |
| | | EP | 3411151 B1 | 16 June 2021 |
| | | ES | 2881342 T3 | 29 November 2021 |
| | | JP | 6938517 B2 | 22 September 2021 |
| | | KR | 10-2018-0105225 A | 27 September 2018 |
| | | MX | 2018009250 A | 10 September 2018 |
| | | NZ | 744129 A | 26 March 2021 |
| | | PL | 3411151 T3 | 06 December 2021 |
| | | PT | 3411151 T | 03 August 2021 |
| | | TW | 201728894 A | 16 August 2017 |
| | | US | 11400449 B2 | 02 August 2022 |
| | | US | 2021-0187501 A1 | 24 June 2021 |
| | | WO | 2017-133741 A1 | 10 August 2017 |

Form PCT/ISA/210 (patent family annex) (July 2022)